# FASCICULE DE BREVET EUROPEEN

(11) **EP 0 527 099 B1**
(45) Date de publication et mention de la délivrance du brevet: **13.11.1996**
(21) Numéro de dépôt: 92460024.0
(22) Date de dépôt: 07.08.1992
(51) Int. Cl.: G01N 33/18, B01F 11/00

(54) **Automate de détection de pollution en milieu aqueux mettant en oeuvre un test sur microorganisme.**
Automatische Vorrichtung zur Ermittlung von Verunreinigungen in wässrigen Medien unter Verwendung von eimem Mikroorganismus
Apparatus for automatically detecting pollution in an aqueous medium by testing a microorganism

(30) Priorité: 07.08.1991 FR 9110217
(43) Date de publication de la demande: 10.02.1993
(73) Titulaire: GIE ANJOU-RECHERCHE, F-75008 Paris (FR)
(72) Inventeur: Henriet, Catherine, F-75011 Paris (FR); Levi, Yves, F-78250 Meulan (FR)
(74) Mandataire: Corlau, Vincent

(56) Documents cités:
- EP-A- 0 149 383
- WO-A-85/00890
- DE-U- 9 015 256
- GB-A- 2 005 018
- US-A- 3 226 094
- US-A- 4 146 364
- US-A- 4 893 938
- ISA TRANSACTIONS, vol. 20, no. 1, 1981, Pittsburgh, PA (US); A.A. BULICH et al. , pp. 29-33/

## Description

L'invention concerne le domaine de la détection de pollution en milieu aqueux telle que par exemple la détection de la pollution des cours d'eau ou des rejets et plus particulièrement le domaine des dispositifs automatiques utilisés pour ce type de détection.

Les stations automatiques de détection et d'analyse de pollution, telles que les stations de protection des prises d'eau des unités de production d'eau potable comprennent des dispositifs permettant de contrôler automatiquement en continu un certain nombre de paramètres physico-chimiques tels que la teneur de l'eau analysée en hydrocarbures, en amoniaque ou en métaux lourds. Ces dispositifs permettent d'identifier ces différents types de polluant qualitativement et quantitativement.

En complément, ces stations comprennent également des installations permettant d'obtenir une détection de la pollution même en l'absence d'identification du polluant et munies de moyens d'alarme pouvant être déclenchés lorsqu'une pollution massive, se traduisant par une toxicité aiguë de l'eau analysée, est détectée.

De tels dispositifs mettent en oeuvre des tests sur organismes vivants consistant à observer les modifications du métabolisme de ces organismes en présence de l'eau analysée, pendant un temps déterminé. Les critères biologiques pris en compte pour observer les modifications du métabolisme sont généralement la mort ou toute autre modification pouvant être constatée visuellement. L'intérêt de tels tests réside dans le fait que leur interprétation d'un point de vue sanitaire est immédiate puisqu'ils consistent à observer la réponse d'un organisme vivant à la qualité de son environnement. Le résultat obtenu est donc global et représentatif de toutes les réactions complexes (tels que des effets de synergie ou d'antagonisme) qui ont lieu au sein de l'écosystème aquatique et qui ne peuvent être appréhendées par l'analyse physicochimique.

En étant basés sur un critère non spécifique, c'est-à-dire la toxicité au niveau d'un organisme, ces tests offrent à l'utilisateur la possibilité de dépister un plus grand nombre de pollutions.

Plusieurs types de tests sur organismes vivants ont à cet effet été mis au point. Les plus fréquemment employés, consistent à observer la survie de Brachydanio rerio, de Daphnia magna ("test Daphnies") ou de Colpidium campylum dans un échantillon de l'eau surveillée. La norme AFNOR T90-301 préconise ainsi l'utilisation du microcrustacé Daphnia magna (test Daphnie). Ces tests demandent un temps d'exposition de l'ordre de 24 heures nécessaire pour interpréter de façon fiable la réponse de l'organisme observé à son environnement aquatique.

Cependant, de tels tests présentent l'inconvénient de ne pouvoir être mis en oeuvre dans les installations automatiques de contrôle permanent.

D'autres tests, pouvant être effectués en continu sont donc utilisés. Ainsi un test consiste à placer des truites dans un bac dans lequel circule un courant de l'eau à surveiller. La mort des truites traduisant une pollution aiguë de l'eau est détectée grâce à un dispositif adapté. Ce test n'est toutefois pas fréquemment utilisé du fait de sa faible sensibilité qui le rend inapplicable au contrôle de la pollution des eaux au-delà d'une certaine toxicité.

D'autres tests, utilisant des microorganismes, ont donc été conçus présentant une sensibilité comparable aux tests mettant en oeuvre les daphnées et ayant de surcroît l'avantage de pouvoir être automatisés.

Ce type de tests possède de nombreux atouts, notamment celui de mettre en oeuvre des microorganismes pouvant être stockés sous forme lyophilisée et présentant ainsi des durées de conservation longues.

Un des tests mettant en oeuvre des microorganismes les plus performants consiste à observer l'intensité lumineuse émise par des bactéries luminescentes présentes dans une eau à analyser par rapport à l'intensité lumineuse produite par la même quantité de bactéries luminescentes présente dans une même quantité d'eau de référence. La baisse de l'intensité lumineuse, proportionnelle à la baisse du métabolisme des bactéries, permet d'évaluer la toxicité de l'eau analysée.

On utilise ainsi Phosphobacterium phosphoreum, une bactérie marine dont la propriété est d'émettre naturellement des photons au cours de son métabolisine.

Le test d'inhibition de luminescence bactérienne présente de nombreux avantages par rapport aux autres tests de toxicité notamment au niveau de la rapidité, de la sensibilité et de la fiabilité. En effet, alors que les tests classiques tel que le test Daphnies demande un délai de réponse d'environ 24 h, le test d'inhibition de luminescence bactérienne permet d'obtenir un résultat en moins d'une heure, durée incluant les phases de préparation et de mesure. La sensibilité de ce test présente par ailleurs des performances voisines de celles du test Daphnies et une reproductibilité bien supérieure.

De plus, chaque test est réalisé sur environ 20 000 bactéries. Son résultat offre ainsi une véritable représentativité statistique.

Ce type de test présente par ailleurs l'avantage de pouvoir être mis en oeuvre dans des installations automatisées pouvant fonctionner sans intervention humaine.

Les microorganismes peuvent en effet être stockés sous forme lyophilisée de façon à ce que l'installation dispose d'une réserve de microorganismes lui permettant de fonctionner de façon autonome pendant une durée pouvant aller jusqu'à 7 jours. Ces installations peuvent être munies en conséquence de moyens de télétransmission permettant en cas de détection de pollution de déclencher l'alerte à distance.

L'utilisation de souches lyophilisées présente de plus l'avantage de supprimer les nombreuses contraintes liées à la mise en place d'une culture ou d'un élevage permanent nécessaire par exemple dans le cas du test Daphnies.

L'utilisation de souches de microorganismes sous forme lyophilisées pour effectuer ces tests posent cependant des problèmes liés à leur reconstitution c'est-à-dire l'opération consistant à réactiver le métabolisme des microorganismes, ce métabolisme ayant été suspendu par l'opération de lyophilisation. Lorsque le test est effectué, la souche doit en effet être reconstituée selon des critères permettant de conférer au test une grande reproductibilité. Cette opération de reconstitution se déroule généralement de la façon suivante :
- les ampoules contenant la souche bactérienne lyophilisée conservées à une température de 4°C à 8°C sont ouvertes,
- le liquide de reconstitution, qui peut par exemple être constitué d'eau distillée aditionnée de chlorure de sodium, est introduit dans l'ampoule,
- l'ampoule est refermée et agitée de façon à provoquer le mélange du liquide de reconstitution avec la souche bactérienne lyophilisée ;
- la solution bactérienne ainsi reconstituée peut alors être stockée et présente une activité lui permettant d'être utilisée dans un délai plus ou moins long selon la bactérie mise en oeuvre et de l'ordre de 12 heures pour Phosphobacterium phosphoreum.

On s'est aperçu que cette dernière étape de mélange de la souche bactérienne lyophilisée était déterminante pour la bonne reproductibilité du test. Les travaux qui ont été effectuées sur ce sujet ont conduit à rechercher une homogénéisation du mélange en agitant celui-ci par exemple avec un dispositif vibreur du type VORTEX.

Toutefois, l'utilisation de VORTEX conduit à une destruction d'une partie de la souche bactérienne contenue dans le mélange agitée. Les vibrations d'un tel dispositif n'étant pas absolument reproductibles cette carence de reproductibilité fait varier le taux de destruction de la souche selon les échantillons et influe sur la reproductibilité même du test.

L'objectif de l'invention est de proposer un automate de détection et de quantification de la pollution en milieu aqueux mettant en oeuvre un test sur microorganisme et présentant des moyens permettant de mélanger la souche lyophilisée du microorganisme utilisé à un liquide de reconstitution selon une méthode permettant de conférer au test une reproductibilité élevée.

Un autre objectif de l'invention est de fournir un automate muni de tels moyens ne pouvant détruire, ne serait-ce qu'en partie, la souche bactérienne utilisée.

Ces objectifs ainsi que d'autres sont atteints grâce à l'invention qui a pour objet un automate de détection de pollution en milieu aqueux mettant en oeuvre un test sur microorganisme et comprenant des moyens permettant de reconstituer la souche lyophilisée de ce microorganisme en la mélangeant dans au moins un conteneur à un liquide de reconstitution. Les conteneurs peuvent être constitués par les ampoules placées sous-vide d'air dans lesquelles est conditionnée ladite souche lyophilisée.

L'invention est caractérisée en ce que ledit automate comprend des moyens de retournement dudit conteneur aptes à permettre le mélange de ladite souche avec ledit liquide de reconstitution sans provoquer la destruction même partielle de la souche. On entend dans le présent texte le terme retournement comme le mouvement de rotation totale ou partielle dudit conteneur dans un plan vertical ou sensiblement vertical selon un axe perpendiculaire ou sensiblement perpendiculaire à son axe longitudinal.

Lesdits moyens de retournement sont constitués par un axe solidarisé perpendiculairement à un portoir du ou desdits conteneurs ledit axe étant relié à des moyens permettant de lui appliquer une rotation sur lui-même.

Avantageusement, ledit portoir est muni de moyens de thermorégulation permettant de maintenir constante la température des conteneurs qui y sont placés.

Préférentiellement cette température correspond à la température de conservation du microorganisme.

Egalement préférentiellement lesdits moyens permettant d'appliquer une rotation audit axe sont constitués par un mouvement incluant une bielle.

Avantageusement, lesdits moyens de retournement du ou desdits conteneurs permettent d'inculquer auxdits conteneurs un retournement d'au moins 45° dans les deux sens de retournement.

Egalement avantageusement, ledit axe est équipé d'un support des tuyaux d'amenée du fluide de thermorégulation dans lesdits conteneurs.

D'une façon intéressante, ledit support possède une configuration en spirale permettant d'enrouler lesdits tuyaux d'amenée autour dudit axe sans gêner la rotation de celui-ci.

Bien que l'invention puisse être mise en oeuvre pour tous les types d'automate de détection de pollution utilisant un test sur microorganisine préalablement lyophilisé, elle concerne préférentiellement un automate mettant en oeuvre un test consistant à détecter la chute de l'intensité lumineuse correspondant à une baisse du métabolisme de bactéries luminescentes sous l'action d'un effet toxique. Dans ce type de test la mesure de l'intensité lumineuse est en effet effectuée grâce à un photomultiplicateur. Le mélange souche lyophilisée-liquide de reconstitution doit donc être effectué de façon extrêmement reproductible dans le but de ne pas influer sur l'opération menée par le photomultiplicateur.

L'invention, ainsi que les différents avantages qu'elle présente seront plus facilement compris grâce à l'exemple non limitatif de réalisation qui va suivre en référence aux dessins dans lesquels :
- la figure 1 représente un schéma global des différents éléments constitutifs d'un analyseur permettant de tester la toxicité aiguë d'une eau par inhibition de la luminescence bactérienne ;
- la figure 2 représente un diagramme traduisant le principe du test par inhibition de la luminescence bactérienne ;
- la figure 3 représente un graphe tel qu'obtenu grâce à l'analyseur selon la figure 1;
- la figure 4 représente une vue de dessus d'une partie de l'analyseur selon la figure 1 ;
- la figure 5 représente une vue de face du mouvement autorisant le mélange de la souche lyophilisée avec le liquide de reconstitution.

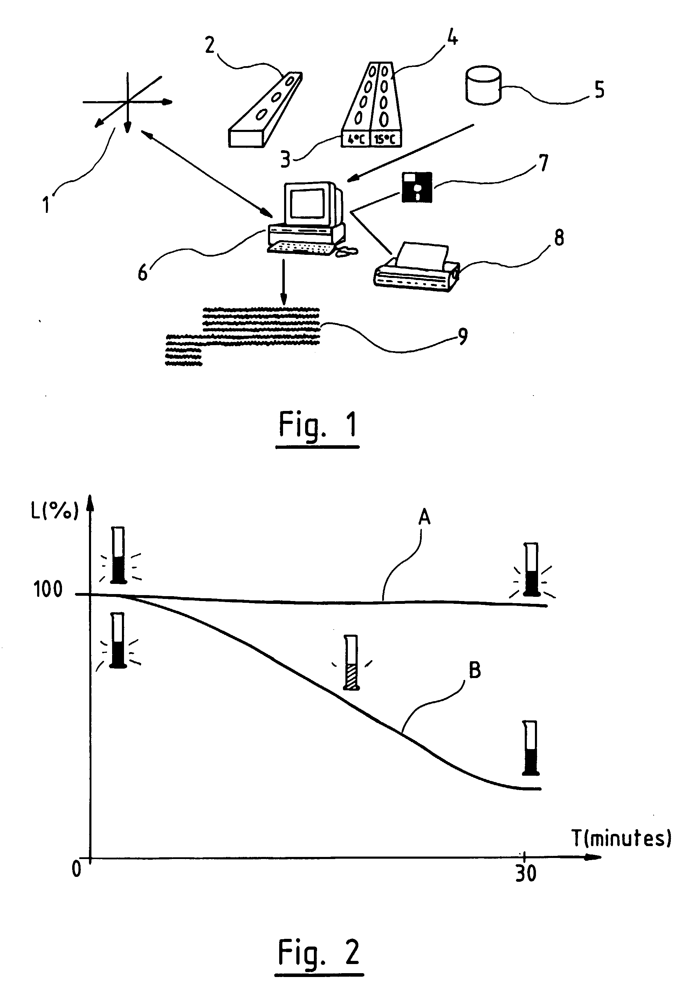

Selon la figure 1, un automate de détection de la pollution en milieu aqueux pouvant être utilisé pour le contrôle de la toxicité des eaux de surface, des effluents industriels, des eaux d'infiltration ou encore des rejets domestiques, comprend :
- un bras manipulateur pouvant déplacer une aiguille selon trois axes X, Y, Z et équipé d'une micro seringue (1) ;
- un compartiment réfrigéré à 4°C pour la conservation des ampoules contenant la souche bactérienne lyophilisée et la reconstitution de la souche bactérienne par addition du liquide de reconstitution (2) ;
- un compartiment réfrigéré pour la conservation des solutions de bactéries reconstituées (3) ;
- un compartiment thermorégulé à la température de travail pour la préparation et la mise en température des échantillons à tester et de l'eau de référence (4) ;
- une unité de mesure incluant un photomultiplicateur et permettant de mesurer l'émission lumineuse des bactéries en présence des substances contenues dans l'eau surveillée par rapport à l'émission lumineuse des bactéries d'un échantillon témoin (5) ;
- un microordinateur (6) fonctionnant grâce à un programme (7) gérant les différents mouvements du bras manipulateur, permettant le calcul de l'inhibition de la bioluminescence, le stockage des différentes données et l'édition des graphes et résultats sur une imprimante (8) et susceptible de télétransmettre un signal d'alerte à une usine (9). Le programme (7) comporte également des instructions permettant le cas échéant d'ajuster et de contrôler manuellement les différentes étapes du test.

Selon la figure 2, le principe du test utilisé par l'automate de détection représenté à la figure 1 est le suivant.

Une bactérie marine (Photobacterium phosphoreum) est capable d'émettre naturellement une lumière dans des conditions favorables à son métabolisme. Cette bactérie peut être conservée durant une longue période sous forme lyophilisée. En présence d'une substance toxique, les cellules bactériennes diminuent leur émission lumineuse, parfois jusqu'à l'extinction complète de celle-ci. Lors du test deux tubes sont inoculés avec une quantité identiques de bactéries. L'un de ces tubes contient une eau de référence non toxique tandis que l'autre tube contient l'eau à tester. L'évolution de l'émission lumineuse (L) en fonction du temps (T) est alors suivie durant 30mn (courbes A et B) à une température de travail de 15°C. La courbe A traduit dans le temps l'évolution de l'émission lumineuse fournie par les bactéries contenues dans l'échantillon de référence et la courbe B traduit durant le même temps l'évolution de l'émission lumineuse fournie par les bactéries contenues dans l'échantillon contenant l'eau surveillée. Si au bout de 30mn, la différence entre l'intensité lumineuse émise par l'échantillon témoin et l'intensité lumineuse émise par l'échantillon testé est supérieure à un certain seuil l'échantillon testé est considéré comme toxique. Le temps de réponse est directement lié à la toxicité du produit et à sa concentration. La diminution de la bioluminescence dans l'échantillon testée peut donc être, selon les cas, observée dès les premières secondes de la mise en contact de la solution bactérienne avec l'eau polluée ou beaucoup plus tard. Ce test permet ainsi d'obtenir une réponse quantitative traduisant le degré de toxicité.

La simplicité et la rapidité de la méthode confèrent à ce test des avantages indéniables qui expliquent que son champ d'application se soit rapidement élargi au contrôle de pollution des eaux - continentales et marines - ainsi qu'à l'étude de la toxicité aigue des produits chimiques.

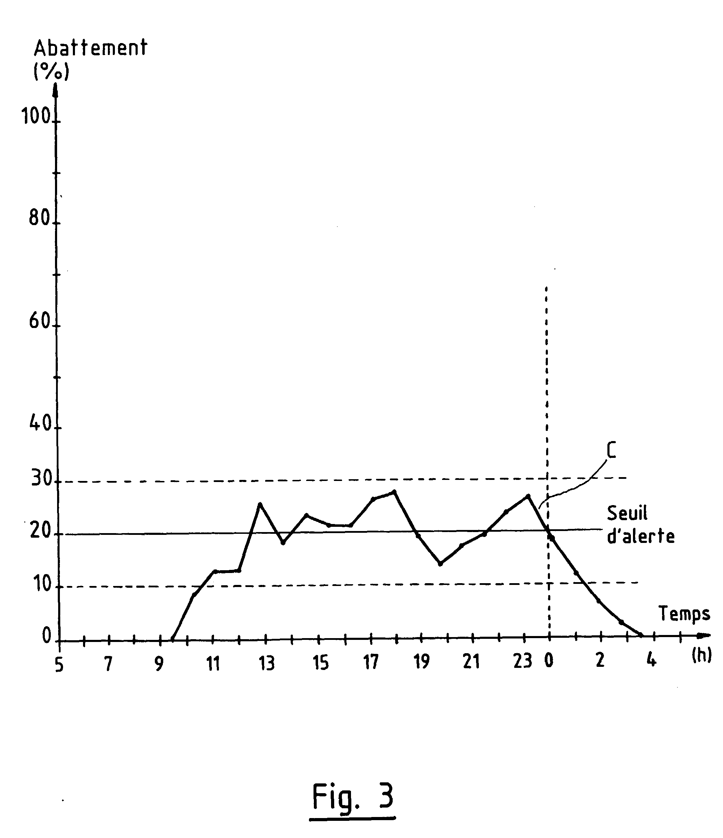

Selon la figure 3, l'analyseur selon la figure 1 a été utilisé pour tester la toxicité d'une eau de rivière. La courbe C traduit l'évolution dans le temps de l'abattement de la bioluminescence des bactéries mises en présence de cette eau, c'est-à-dire la différence d'émission lumineuse constatée après 30mn entre l'échantillon testé, contenant l'eau surveillée et l'échantillon de référence. Le test a été effectué toutes les heures durant 24 heures. La courbe C de résultats laisse apparaître un dépassement du seuil d'alerte entre 13 h et 19 h d'une part et entre 21 h 30 et 0 h d'autre part. L'alerte peut être visualisée à l'écran du microordinateur 3 puis télétransmise à une unité de production d'eau potable située en aval de la station ou est installée l'automate.

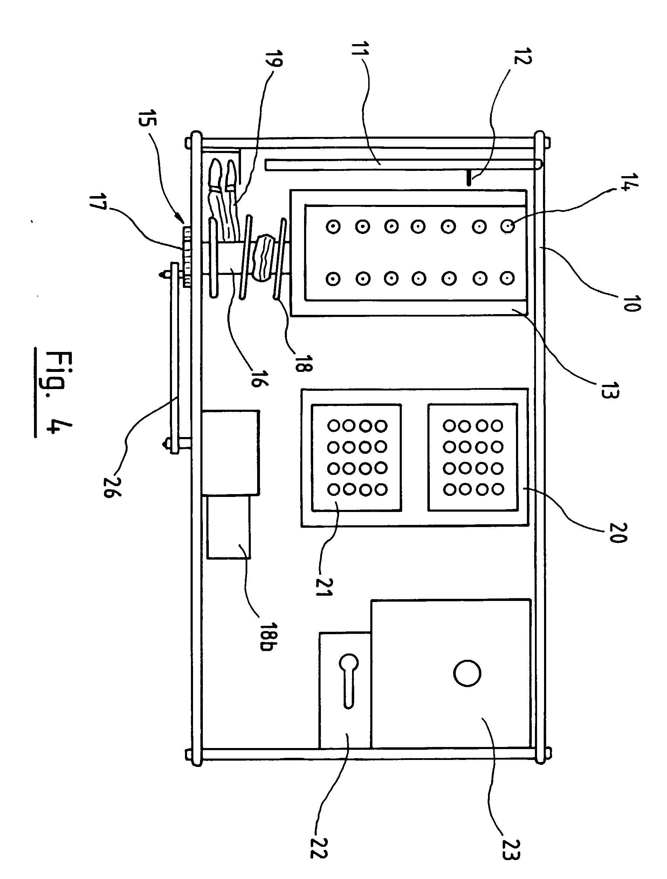

Selon la figure 4 l'analyseur comprend un bâti 10 sur lequel est monté un bras manipulateur 11 muni d'une aiguille 12 permettant de déplacer celle-ci selon trois axes. Le bras manipulateur est relié à une microseringue non représentée sur le schéma permettant la manipulation des différents liquides nécessaires au test. Un portoir 13 thermorégulé à 4°C est monté à l'intérieur de ce bâti 10 et présente des réceptacles dans lesquels peuvent être disposées des ampoules 14 contenant une quantité déterminée d'une souche lyophilisée de Phosphobacterium phosphoreum. Le portoir 13 est par ailleurs équipé de moyens 15 de retournement constitués par un axe 16 qui lui est solidarisé perpendiculairement, ledit axe 16 pouvant être mis en rotation grâce à un mouvement 17 commandé par un moteur 18b. L'axe 16 est équipé d'un support 18 possédant une configuration en spirale. Ce support 18 permet d'accueillir les tuyaux d'amenée 19 du liquide de refroidissement du système de régulation du portoir 13 et de ne pas gêner la circulation de ce liquide lors de la rotation de l'axe 16.

L'analyseur comprend également un portoir 20 de stockage intermédiaire de la suspension bactérienne reconstituée équipé de moyen de thermorégulation permettant de maintenir sa température à 4°C.

Un troisième portoir 21 permet d'accueillir des tubes au niveau desquels sont amenées à température les eaux à tester et les témoins. A cet effet, l'eau à surveiller est amenée continûment à disposition dans un puits 22.

Une unité de mesure 23 comprenant un photomultiplicateur en regard duquel est disposé un barillet muni de deux tubes destinés à recevoir les suspensions bactériennes avec l'échantillon pour l'une et l'eau de référence pour l'autre. Ce dispositif permet de suivre l'évolution de la bioluminescence de chacune des deux solutions sans autre manipulation que la rotation du barillet.

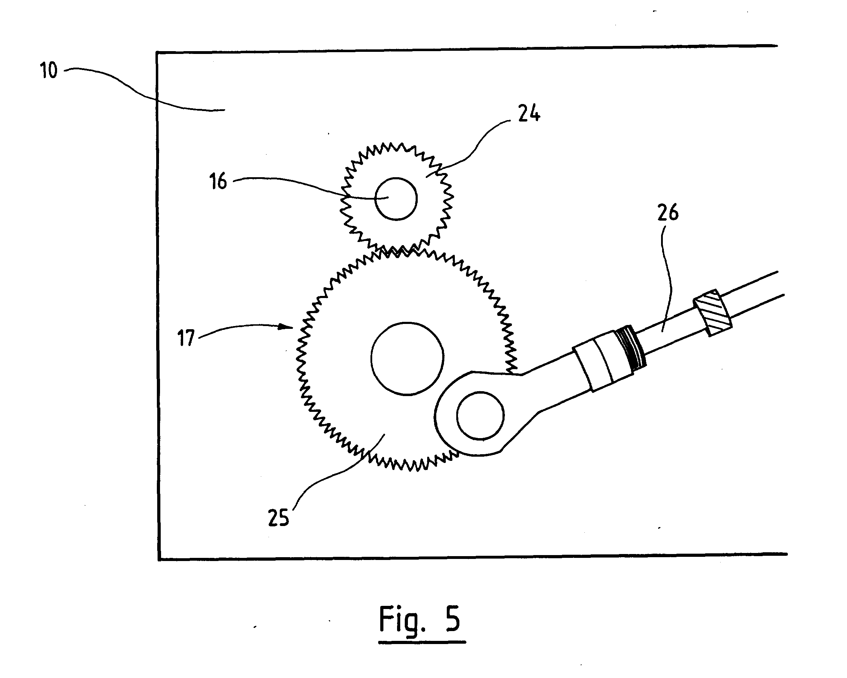

Selon la figure 5 le mouvement 17 permettant de mettre en rotation l'axe 16 comprend une roue dentée 24 solidaire de l'axe 16 combinée à une seconde roue dentée multiplicatrice 25. Une bielle 26 actionnée par le moteur 18 permet de mettre la roue 25 en rotation et ainsi d'induire la mise en rotation de l'axe 16 par transmission de la roue dentée 24.

Le fonctionnement du dispositif est le suivant.

Le portoir 13 est chargé de quatorze ampoules 14 contenant chacune un lyophilisat de Phosphobacterium phosphoreum. Ces ampoules 14 sont fermées par un bouchon pouvant être percé par l'aiguille 12 actionnée par le bras manipulateur 11. Les portoirs 20 et 21 sont chargés chacuns de 16 tubes vides. Lors de l'utilisation du dispositif, le bras 11 se déplace de façon à positionner l'aiguille 12 sur une des ampoules 14 et perfore le bouchon de ladite ampoule de façon à y injecter 2 ml du liquide de reconstitution constitué par une solution de chlorure de sodium à 2 % dans de l'eau distillée. La salification du liquide de reconstitution est en effet nécessaire pour équilibrer la pression osmotique. Le bras manipulateur 11 retire alors l'aiguille 12 de l'ampoule. Le moteur 18 autorise ensuite la mise en rotation de l'axe 16 grâce à un mouvement 17 et à la bielle 26. La mise en rotation de l'axe 16 provoque le retournement complet du portoir 13 dans le premier sens de retournement puis dans le second sens de retournement.

Cette opération permet de provoquer le mélange de la souche lyophilisée avec le liquide de reconstitution dans des conditions douces non susceptibles de provoquer la destruction même partielle de la souche. La population bactérienne dans le liquide de reconstitution est alors de 10⁸ par ml.

Le bras manipulateur 11 se positionne ensuite au niveau de l'ampoule 14 dans laquelle le mélange a été effectué de façon à permettre à l'aiguille de prélever le contenu de celle-ci. Ce contenu est ensuite acheminé vers l'un des tubes du portoir 20. Le portoir 20 étant thermorégulé à 4°C ce tube permet de stocker une quantité de réactif pouvant être utilisée durant 12 heures.

Le bras manipulateur 11 effectue ensuite la préparation des eaux en vue du test dans deux tubes du portoir 21. 900 µl d'échantillon prélevés au niveau du puits 22 sont élevés au titre de 2% à l'aide de 100 µl de NaCl à 20%. 900 µl d'eau distillée sont amenés à 2% à l'aide de 100 µl de NaCl à 20% pour constituer le témoin. Les deux eaux sont amenées à la température de 15°C à laquelle le test est effectué. Une solution de NaCl est également préparée dans l'un des tubes de l'unité de mesure 23. Le bras manipulateur transfère ensuite 10 µl de réactif bactérien stocké dans le portoir 20 ainsi que 90 µl de NaCl à 2% dans chacun des tubes de l'unité de mesure 23. Après mesure de la luminescence initiale, les 900 µl de l'échantillon et de l'eau de référence provenant de deux tubes du portoir 21 sont transférés dans chacun des tubes de l'unité de mesure. L'opération se poursuit par l'acquisition en parallèle de la bioluminescence de chacun de ces tubes toutes les minutes pendant 30 mn.

L'abattement de la luminescence de l'échantillon contenant l'eau à analyser par rapport à la luminescence de l'échantillon témoin est calculé chaque minute. Les graphes de luminescence et d'abattement obtenus sont affichés sur le moniteur du microordinateur 3 et parallèlement tracés sur l'imprimante 7. Ces graphes peuvent être interprétés par un technicien qui peut donner l'alarme si l'abattement mesuré dépasse un certain seuil. En fin de test, le bras manipulateur 11 procède à la vidange et au rinçage des tubes utilisés avant le début d'un nouveau test.

Le nombre d'ampoules contenant le lyophilisat de Phosphobacterium phosphoreum disposées dans le portoir 14 permettant au dispositif d'avoir une autonomie complète de 7 jours, celui-ci peut fonctionner en l'absence d'une présence humaine pendant toute cette durée. Les dépassements du seuil d'abattement se traduise alors par le déclenchement d'une alarme télétransmise.

En période d'alerte, la fréquence du test peut être accélérée de façon à suivre de façon plus précise l'évolution de cet abattement.

Les aspects du présent exemple de réalisation de l'invention ci-dessus précisés ne sont en aucun cas limitatifs.

## Revendications

1. Automate de détection de pollution en milieu aqueux mettant en oeuvre un test sur microorganisme comprenant des moyens permettant de reconstituer la souche lyophilisée dudit microorganisme en la mélangeant dans au moins un conteneur (14) à un liquide de reconstitution,
caractérisé en ce qu'il comprend des moyens de retournement (15) dudit conteneur (14) aptes à permettre le mélange de ladite souche avec ledit liquide de reconstitution sans provoquer de destruction même partielle de ladite souche, lesdits moyens de retournement (15) étant constitués par un axe (16) solidarisé perpendiculairement à un portoir (13) du ou desdits conteneurs (14) ledit axe (16) étant relié à des moyens permettant de lui appliquer une rotation sur lui-même.

2. Automate de détection de pollution selon la revendication 1 caractérisé en ce que lesdits moyens de retournement (15) du ou desdits conteneurs (14) permettent d'inculquer auxdits conteneurs (14) un retournement d'au moins 45° dans les deux sens de retournement.

3. Automate de détection selon la revendication 1 ou 2 caractérisé en ce que ledit portoir (13) est muni de moyens de thermorégulation permettant de maintenir constante la température du ou des conteneurs (14) qui y sont placés.

4. Automate de détection selon la revendication 3 caractérisé en ce que ladite température correspond à la température de conservation dudit microorganisme.

5. Automate de détection de pollution selon l'une des revendications 1 à 4 caractérisé en ce que lesdits moyens permettant d'appliquer une rotation audit axe sont constitués par une mouvement (17) incluant une bielle (26).

6. Automate de détection de pollution selon l'une des revendications 1 à 5 caractérisé en ce que ledit axe est équipé d'un support (18) des tuyaux d'amenée (19) du fluide de thermorégulation dudit portoir (13).

7. Automate de détection de pollution selon la revendication 6 caractérisé en ce que ledit support (18) possède une configuration en spirale permettant d'enrouler lesdits tuyaux d'amenée (19) autour dudit axe (16) sans gêner la rotation de celui-ci.

8. Automate de détection de pollution selon l'une des revendications 1 à 7 caractérisé en ce que ledit test consiste à détecter la chute de l'intensité lumineuse correspondant à une baisse du métabolisme de bactéries luminescentes sous l'action d'un effet toxique.

9. Automate de détection de pollution selon la revendication 8 caractérisé en ce que ladite bactérie luminescente utilisée pour le test est Photobacterium phosphoreum.

## Claims

1. Automaton for detecting pollution in an aqueous medium, employing a test on a microorganism, comprising means making it possible to reconstitute the freeze-dried strain of the said microorganism by mixing it in at least one container (14) with a reconstitution liquid, characterised in that it comprises means (15) for overturning the said container (14), which are capable of allowing the mixing of the said strain with the said reconstitution liquid without causing even partial destruction of the said strain, the said overturning means (15) consisting of an axle (16) rigidly attached perpendicularly to a rack (13) of the said container or containers (14), the said axle (16) being connected to means allowing it to be rotated on itself.

2. Automaton for detecting pollution according to Claim 1, characterised in that the said means (15) for overturning the said container or containers (14) make it possible to impart to the said containers (14) overturning through at least 45° in both directions of overturning.

3. Detection automaton according to Claim 1 or 2, characterised in that the said rack (13) is fitted with thermoregulation means making it possible to keep the temperature of the container or containers (14) which are placed therein constant.

4. Detection automaton according to Claim 3, characterised in that the said temperature corresponds to the temperature of preservation of the said microorganism.

5. Automaton for detecting pollution according to one of Claims 1 to 4, characterised in that the said means making it possible to rotate the said axle consist of a gear mechanism (17) including a connection rod (26).

6. Automaton for detecting pollution according to one of Claims 1 to 5, characterised in that the said axle is equipped with a support (18) for the feed pipes (19) of the fluid for thermoregulation of the said rack (13).

7. Automaton for detecting pollution according to Claim 6, characterised in that the said support (18) has a spiral configuration allowing the said feed pipes (19) to be wound around the said axle (16) without obstructing its rotation.

8. Automaton for detecting pollution according to one of Claims 1 to 7, characterised in that the said test consists in detecting the drop in light intensity corresponding to a fall in the metabolism of the luminescent bacteria under the influence of a toxic effect.

9. Automaton for detecting pollution according to Claim 8, characterised in that the said luminescent bacterium used for the test is Photobacterium phosphoreum.

## Patentansprüche

1. Automat zur Feststellung von Schadstoffen in wäßriger Umgebung durch Anwendung von Versuchen an Mikroorganismen, welcher Mittel enthält, die die Wiederherstellung des lyophilisierten Stamms der Mikroorganismen durch Vermischung mit einer Wiederherstellungsflüssigkeit in mindestens einem Behälter (14) gewährleisten,
dadurch gekennzeichnet, daß er Kippvorrichtungen (15) für den Behälter (14) enthält, welche die Vermischung des Stamms mit der Wiederherstellungsflüssigkeit gewährleisten, ohne eine auch nur teilweise Vernichtung des Stammes zu verursachen,
daß die Kippvorrichtungen (15) eine Welle (16) enthalten, welche senkrecht mit einer Trägereinrichtung (13) für den oder die Behälter verbunden ist, wobei die Welle (16) mit einer Vorrichtung verbunden ist. die Rotation der Welle (16) um die eigene Achse ermöglicht.

2. Automat zur Feststellung von Schadstoffen gemäß Anspruch 1, dadurch gekennzeichnet, daß die Kippvorrichtungen (15) für den oder die Behälter (14) ein Kippen der Behälter (14) um mindestens 45° in beide Kipprichtungen gewährleisten.

3. Automat zur Feststellung von Schadstoffen gemäß Anspruch 1 oder 2.
dadurch gekennzeichnet, daß die Trägereinrichtung (13) über Temperaturregulierungssysteme verfügt, welche die Konstanthaltung der Temperatur der dort untergebrachten Behälter (14) gewährleisten.

4. Automat zur Feststellung von Schadstoffen gemäß Anspruch 3,
dadurch gekennzeichnet, daß die Temperatur der Konservierungstemperatur der Mikroorganismen entspricht.

5. Automat zur Feststellung von Schadstoffen gemäß einem der Ansprüche 1 bis 4,
dadurch gekennzeichnet, daß die Mittel zum Versetzen der Welle in eine Rotationsbewegung aus einem Mechanismus (17) bestehen, der eine Kurbelstange (26) enthält.

6. Automat zur Feststellung von Schadstoffen gemäß einem der Ansprüche 1 bis 5,
dadurch gekennzeichnet, daß die Welle mit einer Halterung (18) für die Zufuhrschläuche (19) der Temperaturregulierungsflüssigkeit der Trägereinrichtung (13) versehen ist.

7. Automat zur Feststellung von Schadstoffen gemäß Anspruch 6,
dadurch gekennzeichnet, daß die Halterung (18) eine spiralförmige Konfiguration besitzt, welche das Umwickeln der Zufuhrschläuche (19) um die Welle (16) ermöglicht, ohne deren Rotation zu behindern.

8. Automat zur Feststellung von Schadstoffen gemäß einem der Ansprüche 1 bis 7,
dadurch gekennzeichnet, daß der Versuch in der Feststellung der Leuchtkraftabnahme besteht, welche einem Stoffwechselrückgang der Leuchtbakterien unter dem Einfluß toxischer Stoffe entspricht.

9. Automat zur Feststellung von Schadstoffen gemäß Anspruch 8,
dadurch gekennzeichnet, daß es sich bei den für den Versuch angewandten Leuchtbakterien um Photobacterium Phosphoreum handelt.
